# EUROPEAN PATENT SPECIFICATION

(11) **EP 2 872 220 B1**
(45) Date of publication and mention of the grant of the patent: **07.11.2018**
(21) Application number: 13740481.0
(22) Date of filing: 16.07.2013
(51) Int. Cl.: A61Q 15/00, A61K 8/81, A61K 8/27, A61Q 17/00

(54) **DEODORANT METHODS**
DESODORIERUNGSVERFAHREN
PROCÉDÉS DE DÉSODORISATION

(30) Priority: 16.07.2012 US 201261671938 P
(43) Date of publication of application: 20.05.2015
(73) Proprietor: The Procter & Gamble Company, Cincinnati, OH 45202 (US)
(72) Inventor: LANZALACO, Anthony, Charles, Cincinnati, OH 45202 (US); STARK, Cynthia, Marie, Cincinnati, OH 45202 (US); EYLEM, Cahit, Cincinnati, OH 45202 (US); HEAPE, Andrea, Kay, Cincinnati, OH 45202 (US); CETTI, Jonathan, Robert, Cincinnati, OH 45202 (US)
(74) Representative: Sauvaître, Thibault Bruno
(86) International application number: PCT/US2013/050599
(87) International publication number: WO 2014/014861

(56) References cited:
- WO-A1-97/32480
- WO-A1-2012/078208
- US-A- 4 675 178
- US-A1- 2009 226 394
- US-B1- 6 261 581

## Description

### FIELD

Deodorant compositions comprising polyvinylamine polymers and/or metalated malodor control polymers and methods thereof.

### BACKGROUND

There are currently many deodorant products on the market. These products often utilize high perfume levels in order to disguise body odor. These high perfume levels can be irritating to the skin and they do nothing to prevent the formation of body odor. Another avenue of reducing odor is to utilize odor neutralizers. These materials can act in several ways. For example, they can react with odor causing materials causing a chemical change which reduces odor (like reacting with odor causing aldehydes to remove them from skin) or they can complex with odor causing materials preventing them from being expressed. Like perfume level, these types of materials do nothing to prevent the formation of odor itself. At least some body odor is the result of microorganisms on the skin which break down sweat to produce the smell that is associated with body odor. Thus, there remains a need for deodorant compositions which neutralize body odor by targeting the microorganisms that create it, including those odor causing bacteria that reside in the hair follicles of the underarm.

### SUMMARY

Use of a malodor control polymer comprising a polyvinyl amine polymer, wherein the polyvinyl amine polymer comprises a copolymer comprising 95% mol vinyl monomer and 5% mol vinylformamide monomer, in a cosmetic composition for reducing the odor caused by the growth of bacteria on skin, wherein the bacteria is C. mucifaciens.

A method of reducing underarm bacteria, comprising: applying a roll-on deodorant composition comprising from about 75% to about 90%, by weight of the composition, of water; from about 1% to about 30%, by weight of the composition, of an emollient; from about 0.01% to about 5% of a solubilizer; and from about 0.5% to about 5%, by weight of the composition, of a malodor control polymer comprising a polyvinyl amine polymer, wherein the polyvinyl amine polymer comprises a copolymer comprising 95% mol vinyl monomer and 5% mol vinylformamide monomer and wherein the bacteria comprise C. mucifaciens.

A method of reducing underarm bacteria, comprising: applying a spray deodorant composition comprising from about 15% to about 45%, by weight of the composition, of water; from about 15% to about 45%, by weight of the composition, of alcohol; from about 0.5% to about 5%, by weight of the composition, of a malodor control polymer comprising a polyvinyl amine polymer, wherein the polyvinyl amine polymer comprises a copolymer comprising 95 % mol vinyl monomer and 5% mol vinylformamide monomer, and a propellant, wherein the bacteria comprise C. mucifaciens.

### BREIF DESCRIPTION OF THE DRAWINGS

Fig. 1 is a graph showing the antibacterial activity of an inventive leave-on deodorant composition comprising a metalated malodor control polymer versus a control composition (no metalated malodor control polymer) at baseline and post treatment;
Fig. 2 is a graph showing the antibacterial activity of an inventive leave-on deodorant composition comprising a malodor control polymer and of an inventive leave-on deodorant composition comprising a metalated malodor control polymer at baseline and post treatment;
Fig. 3 is a graph showing the antibacterial activity of a leave-on deodorant composition comprising zinc phenolsulfonate (ZPS) and of an inventive leave-on deodorant composition comprising a metalated malodor control polymer at baseline and post treatment;
Fig. 4 is a graph showing 12 hour self assessed odor of a placebo composition versus an inventive leave-on deodorant composition comprising a metalated malodor control polymer;
Fig. 5 is a graph showing 24 hour self assessed odor of a placebo composition versus an inventive leave-on deodorant composition comprising a metalated malodor control polymer;
Fig. 6 is a graph showing 12 hour self assessed odor of an inventive leave-on deodorant composition comprising a malodor control polymer versus an inventive leave-on deodorant composition comprising a metalated malodor control polymer;
Fig. 7 is a graph showing 24 hour self assessed odor of an inventive leave-on deodorant composition comprising a malodor control polymer versus an inventive leave-on deodorant composition comprising a metalated malodor control polymer;
Fig. 8 is a graph showing 12 hour self assessed odor of an inventive leave-on deodorant composition comprising a metalated malodor control polymer versus a zinc phenolsulfonate containing leave-on deodorant product; and
Fig. 9 is a graph showing 24 hour self assessed odor of an inventive leave-on deodorant composition comprising a metalated malodor control polymer versus a zinc phenolsulfonate containing leave-on deodorant product.

### DETAILED DESCRIPTION

This application claims the benefit of U.S. Provisional Application Serial No. 61/671, 938.

The composition is designed to deliver genuine malodor reduction and not function merely by using perfume to cover up or mask odors. A genuine malodor reduction provides a sensory (e.g. self assessed body odor) malodor reduction and/or analytically measurable (e.g. hair pluck method) bacteria control. Thus, if the composition delivers a genuine malodor reduction, the composition will reduce or prevent the formation of at least some of the malodors on the body.

"Deodorant composition" as used herein refers to a composition that is applied to at least a portion of the body, which is used to combat body odor.

"Leave-on" as used herein refers to a composition that is designed to be applied to at least a portion of the body and then left on that portion of the body.

### I. Deodorant Compositions

As noted above, there are several technical approaches to reducing body odor after the odor causing material has formed, like the use of increased perfume levels and odor neutralizers. Another approach is to target the organisms, like bacteria, that contribute to body odor by using an antibacterial agent. Some examples of bacteria on the skin that can cause body odor are S. *epidermidis* and *C*. *mucifaciens.*

While polyvinyl amine polymers (PVam) are sometimes used as odor neutralizers, the present inventors have surprisingly discovered that polyvinyl amine polymers and metalized polyvinyl amine polymers can also exert an antimicrobial effect against key odor causing bacteria. See, for example, Table 1 where the Minimal Inhibitory Concentration required to achieve 100% inhibition in bacterial growth (MIC100) is shown. Relative to a control formulation that did not contain either the polymer or the metalized polymer, roll-on formulations containing either PVam (Lupamin® 1595) alone or metalized (combined with zinc chloride), exhibited strong antimicrobial activity against known odor causing bacteria S. *epidermidis* and *C*. *mucifaciens.*

**Table 1**

| Formulation | Serial Dilution Required to Drop Below 100% Inhibition of *S. epidermidis* Growth | Serial Dilution Required to Drop Below 100% Inhibition of *C*. *mucifaciens* Growth |
|---|---|---|
| Roll On Control | < 1:10 | <1:10 |
| Roll On + 0.75% ZnCl₂ | 1:40 | 1:80 |
| Roll On + 2% active PVam (Lupamin® 1595) | > 1:5120 | > 1:5120 |
| Roll On + 2% active PVam (Lupamin® 1595) + 0.75% ZnCl₂ | > 1:5120 | > 1:5120 |

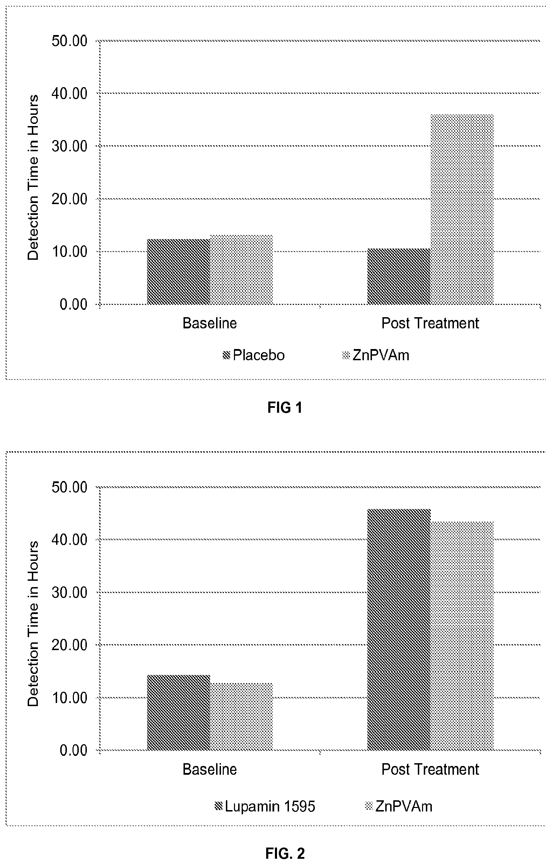

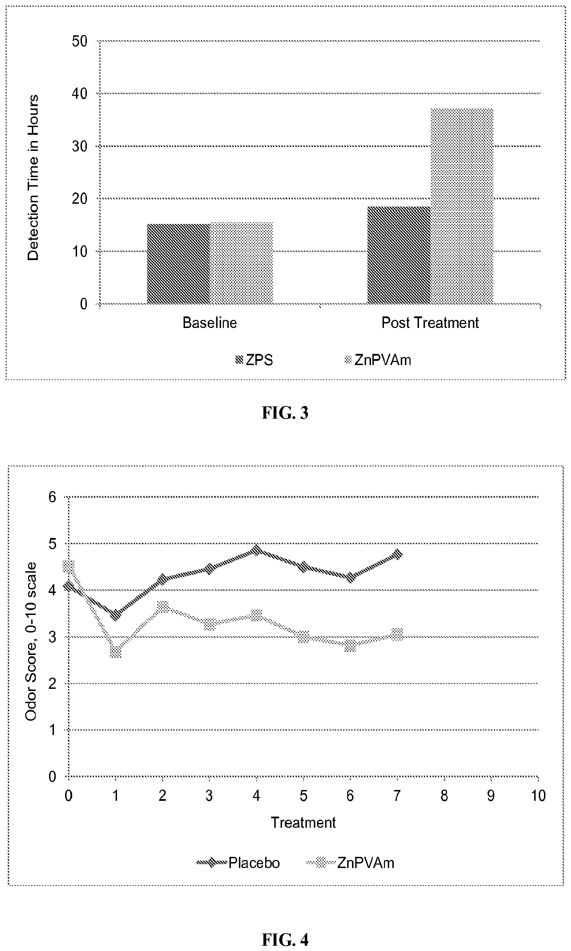

This can also be seen in FIG 1, where the metalized polyvinylamine polymer exhibited strong antibacterial activity when compared to baseline and a control; in FIG. 2, where the polyvinylamine polymer alone and when metalized exhibited strong antibacterial activity; and in FIG. 3 where the metalized polyvinylamine polymer exhibited superior antibacterial activity compared to that of zinc phenolsulfonate (ZPS) .

In reducing body odor, an antimicrobial mechanism has the advantage over odor neutralizing approaches in that antibacterial disruption of the odor forming ecosystem provides long lasting odor protection as the bacteria require time to rebound to their maximum odor forming potential. In contrast, successful odor control using odor neutralization requires the presence of the odor neutralizing agent in sufficient amount to act against the odorants as they appear on the skin. Since odor generation can occur at any time between product applications, this necessitates a high degree of product substantivity well beyond what is observed for current commercial odor control products. Net, approaches to odor control that utilize the chemical removal of odor substances will be limited by how much odor "neutralizing" material remains on the skin at the point in which a bacterially mediated odor burst occurs. Given that topically applied products are rapidly diminished on the skin due to loss onto clothing and being washed away by sweating, it is unlikely that an odor neutralizing approach can maintain sufficient potency over extended periods of time.

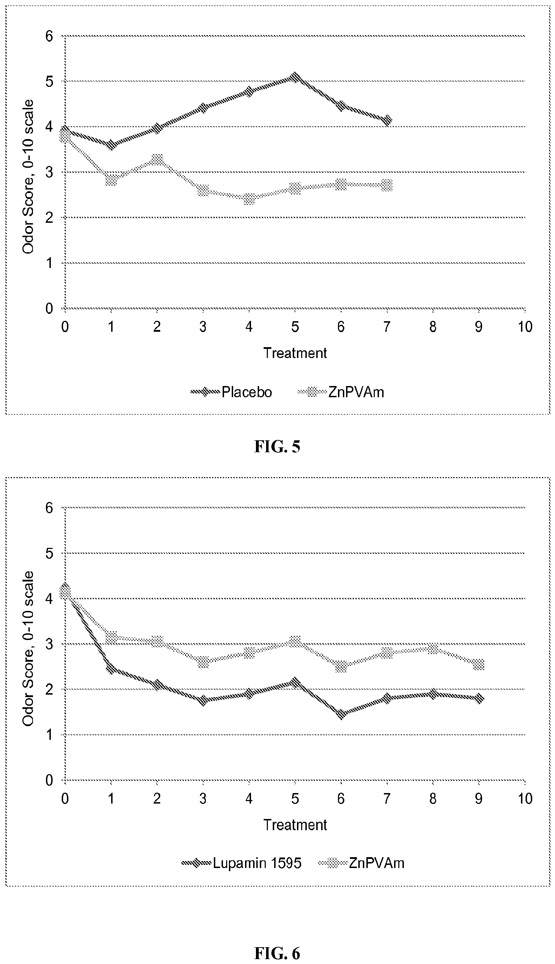

In addition to the significant antibacterial activity, products containing either a polyvinylamine polymer or a metalized polyvinylamine polymer also showed significant antibody odor efficacy in self assessed odor clinical trials. For self assessed body odor, subjects in a study are asked to sniff under each underarm twice daily at 12 and 24 hours post product application and record the level of odor on a scale from 0-10. As can be seen in FIGS. 4 and 5, a product containing a metalized PVam polymer had superior anti-odor efficacy at both 12 & 24 hours following application versus a control product formulation that did not contain the metalized PVam polymer. Odor differences of up to 2 units were observed. In our experience an odor difference of 0.5 units is generally considered consumer noticeable.

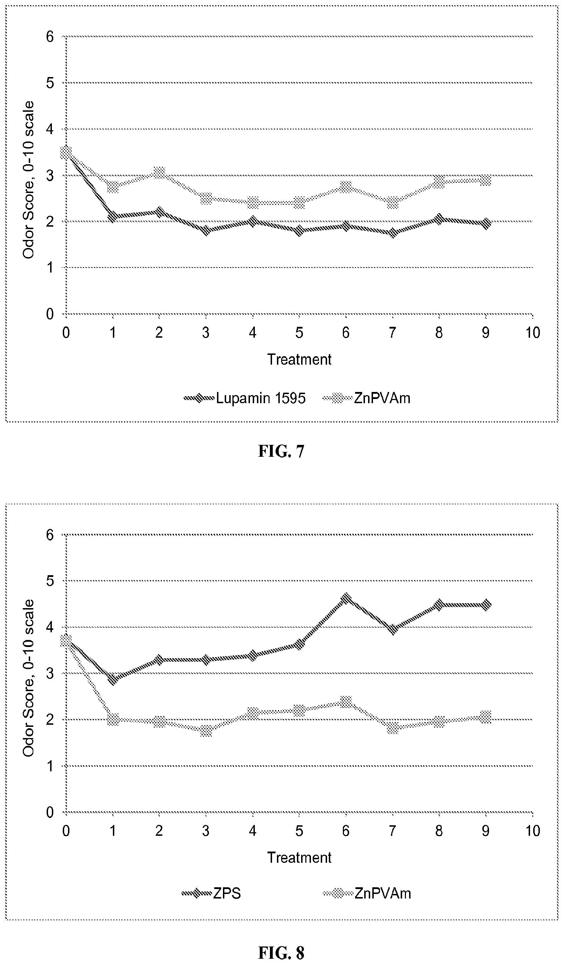

Strong self assessed odor control was also seen for both a product containing a polyvinylamine polymer (Lupamin® 1595) and a product containing a metalized polyvinylamine polymer (Lupamin® 1595 + ZnCl₂). At both 12 and 24 hours following product application both the PVam and metalized PVam formulations exhibited strong reductions in baseline odor scores. This increased level of odor protection was maintained over the course of the study. Interestingly, the polyvinylamine polymer product also showed improved odor control over the metalized polyvinylamine polymer product beginning at treatment 3 at both 12 and 24 hours. Odor differences of up to 1.0 unit were observed (FIGS. 6 and 7).

In addition to the basic antibacterial activity of the polyvinyl amine polymers, it is also surprising to note that these polymers remained biologically active at the underarm skin surface and within underarm hair follicles following their application. Polymeric materials such as Lupamin, which are of relatively high molecular weight (> 2,000 Daltons) often possess poor bioactivity on skin. Such materials historically have difficulty in diffusing into the ecological niches - such as skin crevices and hair follicles - that odor causing bacteria favor. The inability of these larger molecules to fully reach the odor causing bacteria can produce weaker clinical odor control then would be predicted by their in vitro antimicrobial activity. Thus, Lupamin possesses an unexpectedly high level of antibacterial potency against odor forming bacteria and is able to translate this antibacterial effect into strong body odor control by its equally unexpected ability to target and control the odor causing bacteria residing in the hard to treat areas of the skin which are usually unaccessible to a polymer of its size.

A leave-on deodorant composition for reducing body malodor by reducing bacteria in the underarm comprises an effective amount of a malodor control polymer.

### A. Malodor Control Polymers

The composition includes a malodor control polymer. Malodor control polymers are water soluble and are formed from a polyamine polymer having a primary amine group. The malodor control polymers are referred to as polyvinylamine (PVam). The composition may comprise from about 0.01% to about 20%, by weight of the composition, of the malodor polymer. The composition may also comprise from about 0.5%, about 1.0%, about 1.5%, about 2%, about 3%, about 5%, to about 2.0%, about 3.0%, about 5%, about 10%, about 15%, about 20%, or any combination thereof, by weight of the composition, of the malodor control polymer. While these polymers often come in solution form, the amounts of polymer, are by active percentage, not percentage of raw material.

A PVam is a linear polymer with pendent, primary amine groups directly linked to the main chain of alternating carbons. PVams are manufactured from hydrolysis of poly(N-vinylformamide) (PVNF) which results in the conversion of formamide units to amino groups as described by the following formula (I1a): 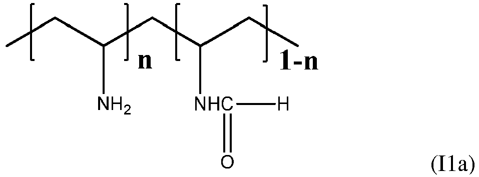 where n is a number from 0.1 to 0.99 depending on the degree of hydrolysis. For instance, in 95% hydrolyzed PVam polymer, n will be 0.95 while 5% of the polymer will have formamide units.

It has been found that a high degree of hydrolysis of PVam increases the resulting polymer's ability to mitigate the odors.

PVams that can be hydrolyzed may have an average molecular weight (MW) of 5,000 to 350,000. Suitable hydrolyzed PVams are commercially available from BASF. Some examples include Lupamin® 9095, 9030, 9010, 5095, and 1595.

### B. Metal Coordinated Complexes

The composition may include a malodor control polymer that is a metal coordinated complex, described herein as a metalated polymer. The composition may comprise from about 0.01% to about 20%, by weight of the composition of the metalated malodor polymer. The composition may also comprise from about 0.5%, about 1.0%, about 1.5%, about 2%, about 3%, about 5%, to about 2.0%, about 3.0%, about 5%, about 10%, about 20%, or any combination thereof, by weight of the composition, of the metalated malodor polymer. The polymer amounts are by percentage of active in the composition, not raw material.

The metalated polymer comprises a metal and any malodor control polymer disclosed herein (i.e. polyvinylamine polymers). Metal coordination may improve the odor neutralization of a malodor control polymer. Metal coordination might also provide reduction of malodor from microbial sources. Suitable metals that coordinate with such polymers include zinc, copper, silver, and mixtures thereof. Suitable metals also include Na, K, Ca, Mg, and non-transition metals, including Sn, Bi, and Al.

Metals that are not coordinated to a polymer may deliver some malodor control using highly ionizable water soluble salts such as zinc chloride or silver nitrate. But, such metals present drawbacks in aqueous formulations. Zinc ions and silver ions have the ability to form insoluble salts with nucleophilic compounds such as valeric acid, skatole, hydrogen sulfide, mercaptan, and like compounds that are typically the cause of environmental malodor. However, zinc chloride aqueous solutions, over time, tend to form insoluble oxychlorides and hydroxides that have low water solubility. As a result, aqueous formulations containing zinc chloride are traditionally kept below pH 4.5 in order to avoid the formation of these insoluble salts that result in cloudy formulations. Just like zinc salts, silver compounds suffer from pH stability, formation of insoluble salts with anions typically present in water. Silver ion, additionally, is very light sensitive and can easily be first reduced to silver metal by photo-reduction process and then oxidized to black silver oxide after lengthy light exposure. For aqueous spray and roll on applications, these issues may be considered detriments.

Coordinating zinc ion or silver ion with polyvinylamine polymers may overcome the limitations described above, resulting in water soluble complexes with a wide range pH stability (e.g. > 4.5). The strength of the metal-nitrogen ligand interaction is influenced by several factors including the microstructure of polymer, functionality of the binding sites, the density of nitrogen ligands in the polymer, steric constraints, electrostatic interactions, pH, pKa of the polymer, and oxidation state, size, and electronic configuration of metal. Unlike traditional chelators, such as ethylenediamine (bi-dentate), ethylenediaminetetraacetic acid, or EDTA (hexa-dentate), polymers can be considered poly-dentate due to high density of binding sites. As a result, the chemical formula of metal coordination complex is highly variable.

Metal coordinated complexes may have a metal / polymer weight ratio from 0.001 and 50, alternatively from 0.001 to 20, alternatively from 0.001 to 15, alternatively from 0.001 to 10, alternatively from 0.005 to 5.0, alternatively from 0.1 to 1.0, alternatively from 0.1 to 0.5, alternatively from 0.001 to 0.01.

Metal polymer coordination complexes can be prepared by reacting suitable metal salts with polyvinylamine polymers containing amine sites. The resulting complex can be represented by a general formula, MxPy; where M is metal, P is an unmodified polyvinylamine polymer, and x and y are integers and dependent on coordination number of metal ion, number of available coordinating sites on the polymer, and pH.

It is believed there is strong competition between the metal ions and protons for the electron pairs on the amine groups of polyvinylamine polymers. This competition is favored for the metal ions at higher pH values, where amine groups are deprotonated and more available for metal binding. It may be assumed that only the non-protonated nitrogen sites of the polymers are active towards the metal ions; then polyvinylamine polymers will have the highest metal binding capacity at high pH levels. Metal ions can coordinate to four to eight ligands. Zinc ion is known to prefer 4-coordinated tetrahedral sites as shown below, while copper ions tend to form octahedral coordinations. Examples of possible zinc polymer structures are shown below. For example, zinc ion can bind to 2 nitrogen units on each PVam. Alternatively, a polymer can fold around zinc ion and utilize four nitrogen to form tetrahedral coordination.

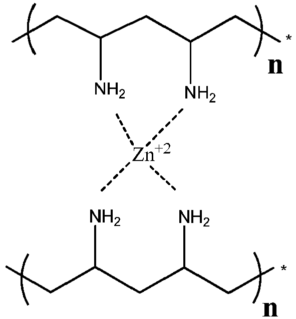

Protonation and metal binding ability of polyvinylamine polymers are also influenced by polymer microstructure. Due to its linear structure, PVams show relatively strong interaction in neighboring ammonium groups on the polymer chain. The deodorant composition as described herein can include a zinc polymer complex. The zinc polymer complex may have a pH of 7. It is believed that at such pH the competition between protonation and metal coordination of amine sites provides a unique coordination environment for zinc. This unique bonding makes the zinc ions readily available for additional interactions with malodor molecules, while preventing the release of zinc ions from the metal coordinated complex.

### C. Water

The deodorant composition may also include water. Water may be present in an amount of about 1% to about 99.5%, about 25% to about 99.5%, about 50% to about 99.5%, about 75% to about 99.5% about 80% to about 99.5%, from about 15% to about 45%, or any combination of the end points and points encompassed within the ranges, by weight of the deodorant composition.

### D. Emollients

Deodorant compositions can comprise an emollient system including at least one emollient, but it could also be a combination of emollients. Suitable emollients are often liquid under ambient conditions. Depending on the type of product form desired, concentrations of the emollient(s) in the deodorant compositions can range from about 1% to about 95%, from about 5% to about 95%, from about 15% to about 75%, from about 1% to about 10%, from about 15% to about 45%, or from about 1% to about 30%, by weight of the deodorant composition.

Emollients suitable for use in the deodorant compositions include, but are not limited to, propylene glycol, polypropylene glycol (like dipropylene glycol, tripropylene glycol, etc.), diethylene glycol, triethylene glycol, PEG-4, PEG-8, 1,2 pentanediol, 1,2 hexanediol, hexylene glycol, glycerin, C2 to C20 monohydric alcohols, C2 to C40 dihydric or polyhydric alcohols, alkyl ethers of polyhydric and monohydric alcohols, volatile silicone emollients such as cyclopentasiloxane, non volatile silicone emollients such as dimethicone, mineral oils, polydecenes, petrolatum, and combinations thereof. One example of a suitable emollient comprises PPG-15 stearyl ether. Other examples of suitable emollients include dipropylene glycol and propylene glycol.

### E. Other Components

The deodorant composition may also include additional ingredients like, for example, propellants, solubilizers, chelants, anti-oxidants, fragrance, encapsulates, odor entrappers, thickener, gelling agents, deodorant actives, other actives, buffering agent, preservatives, dyes, and combinations thereof, etc.

### Propellant

The compositions described herein may include a propellant. Some examples of propellants include compressed air, nitrogen, inert gases, carbon dioxide, and mixtures thereof. Propellants may also include gaseous hydrocarbons like propane, n-butane, isobutene, cyclopropane, and mixtures thereof. Halogenated hydrocarbons like 1,1-difluoroethane may also be used as propellants. Some non-limiting examples of propellants include 1,1,1,2,2-pentafluoroethane, 1,1,1,2-tetrafluoroethane, 1,1,1,2,3,3,3-heptafluoropropane, trans-1,3,3,3-tetrafluoroprop-1-ene, dimethyl ether, dichlorodifluoromethane (propellant 12), 1,1-dichloro-1,1,2,2-tetrafluoroethane (propellant 114), 1-chloro-1,1-difluoro-2,2-trifluoroethane (propellant 115), 1-chloro-1,1-difluoroethylene (propellant 142B), 1,1-difluoroethane (propellant 152A), monochlorodifluoromethane, and mixtures thereof. Some other propellants suitable for use include, but are not limited to, A-46 (a mixture of isobutane, butane and propane), A-31 (isobutane), A-17 (n-butane), A-108 (propane), AP70 (a mixture of propane, isobutane and n-butane), AP40 (a mixture of propane, isobutene and n-butane), AP30 (a mixture of propane, isobutane and n-butane), and 152A (1,1 diflouroethane). The propellant may have a concentration from about 15%, 25%, 30%, 32%, 34%, 35%, 36%, 38%, 40%, or 42% to about 70%, 65%, 60%, 54%, 52%, 50%, 48%, 46%, 44%, or 42%, or any combination thereof, by weight of the total fill of materials stored within the container.

### Deodorant Actives

Suitable optional deodorant actives may include any topical material that is known or otherwise effective in preventing or eliminating malodor associated with perspiration. Suitable deodorant actives may be selected from the group consisting of antimicrobial agents (e.g., bacteriocides, fungicides), malodor-absorbing material, and combinations thereof. For example, antimicrobial agents may comprise cetyl-trimethylammonium bromide, cetyl pyridinium chloride, benzethonium chloride, diisobutyl phenoxy ethoxy ethyl dimethyl benzyl ammonium chloride, sodium N-lauryl sarcosine, sodium N-palmethyl sarcosine, lauroyl sarcosine, N-myristoyl glycine, potassium N-lauryl sarcosine, trimethyl ammonium chloride, sodium aluminum chlorohydroxy lactate, triethyl citrate, tricetylmethyl ammonium chloride, 2,4,4'-trichloro-2'-hydroxy diphenyl ether (triclosan), 3,4,4'-trichlorocarbanilide (triclocarban), diaminoalkyl amides such as L-lysine hexadecyl amide, heavy metal salts of citrate, salicylate, and piroctose, especially zinc salts, and acids thereof, heavy metal salts of pyrithione, especially zinc pyrithione, zinc phenolsulfate, farnesol, and combinations thereof. The concentration of the optional deodorant active may range from about 0.001%, from about 0.01%, of from about 0.1%, by weight of the composition to about 20%, to about 10%, to about 5%, or to about 1%, by weight of the composition.

### Odor Entrappers

The composition may include an odor entrapper. Suitable odor entrappers for use herein include, for example, solubilized, water-soluble, uncomplexed cyclodextrin. As used herein, the term "cyclodextrin" includes any of the known cyclodextrins such as unsubstituted cyclodextrins containing from six to twelve glucose units, especially, alpha-cyclodextrin, beta-cyclodextrin, gamma-cyclodextrin and/or their derivatives and/or mixtures thereof. The alpha-cyclodextrin consists of six glucose units, the beta-cyclodextrin consists of seven glucose units, and the gamma-cyclodextrin consists of eight glucose units arranged in a donut-shaped ring. The specific coupling and conformation of the glucose units give the cyclodextrins a rigid, conical molecular structure with a hollow interior of a specific volume. The "lining" of the internal cavity is formed by hydrogen atoms and glycosidic bridging oxygen atoms, therefore this surface is fairly hydrophobic. The unique shape and physical-chemical property of the cavity enable the cyclodextrin molecules to absorb (form inclusion complexes with) organic molecules or parts of organic molecules which can fit into the cavity. Many perfume molecules can fit into the cavity.

Cyclodextrin molecules are described in US 5,714,137, and US 5,942,217. Suitable levels of cyclodextrin are from about 0.1% to about 5%, alternatively from about 0.2% to about 4%, alternatively from about 0.3% to about 3%, alternatively from about 0.4% to about 2%, by weight of the composition.

### Buffering Agent

The composition may include a buffering agent which may be alkaline, acidic or neutral. The buffer may be used in the composition for maintaining the desired pH. The composition may have a pH from about 3 to about 10, from about 4 to about 9, from about 5 to about 8, from about 6 to about 7, or it may have a pH of about 6.5. One unique aspect of the polyvinyl amine malodor control polymers is its ability to maintain active nitrogen sites at high pH levels which can help enhance the antibacterial effect which comes, at least in part, from the nitrogen sites.

Suitable buffering agents include, for example, hydrochloric acid, sodium hydroxide, potassium hydroxide, and combinations thereof.

The compositions may contain at least about 0%, alternatively at least about 0.001%, alternatively at least about 0.01%, by weight of the composition, of a buffering agent. The composition may also contain no more than about 1%, alternatively no more than about 0.75%, alternatively no more than about 0.5%, by weight of the composition, of a buffering agent.

### Solubilizer

The composition may contain a solubilizer. A suitable solubilizer can be, for example, a surfactant, such as a no-foaming or low-foaming surfactant. Suitable surfactants are nonionic surfactants, cationic surfactants, amphoteric surfactants, zwitterionic surfactants, and mixtures thereof.

Suitable solubilizers include, for example, hydrogenated castor oil, polyoxyethylene 2 stearyl ether, polyoxyethylene 20 stearyl ether, and combinations thereof. One suitable hydrogenated castor oil that may be used in the present composition is polyoxyethylene hydrogenated castor oil.

When the solubilizing agent is present, it is typically present at a level of from about 0.01% to about 5%, alternatively from about 0.01% to about 3%, alternatively from about 0.05% to about 1%, alternatively from about 0.01% to about 0.05%, by weight of the composition.

### Preservatives

The composition may include a preservative. The preservative is included in an amount sufficient to prevent spoilage or prevent growth of inadvertently added microorganisms for a specific period of time, but not sufficient enough to contribute to the odor neutralizing performance of the composition. In other words, the preservative is not being used as the antimicrobial compound to kill microorganisms on the surface onto which the composition is deposited in order to eliminate odors produced by microorganisms. Instead, it is being used to prevent spoilage of the composition in order to increase shelf-life.

The preservative can be any organic preservative material which will not cause damage to fabric appearance, e.g., discoloration, coloration, bleaching. Suitable water-soluble preservatives include organic sulfur compounds, halogenated compounds, cyclic organic nitrogen compounds, low molecular weight aldehydes, parabens, propane diaol materials, isothiazolinones, quaternary compounds, benzoates, low molecular weight alcohols, dehydroacetic acid, phenyl and phenoxy compounds, or mixtures thereof.

Non-limiting examples of commercially available water-soluble preservatives include a mixture of about 77% 5-chloro-2-methyl-4-isothiazolin-3-one and about 23% 2-methyl-4-isothiazolin-3-one, a broad spectrum preservative available as a 1.5% aqueous solution under the trade name Kathon® CG by Rohm and Haas Co.; 5-bromo-5-nitro-1,3-dioxane, available under the tradename Bronidox L® from Henkel; 2-bromo-2-nitropropane-1,3-diol, available under the trade name Bronopol® from Inolex; 1,1'-hexamethylene bis(5-(p-chlorophenyl)biguanide), commonly known as chlorhexidine, and its salts, e.g., with acetic and digluconic acids; a 95:5 mixture of 1,3-bis(hydroxymethyl)-5,5-dimethyl-2,4-imidazolidinedione and 3-butyl-2-iodopropynyl carbamate, available under the trade name Glydant Plus® from Lonza; N-[1,3-bis(hydroxymethyl)2,5-dioxo-4-imidazolidinyl]-N,N'-bis(hydroxy-methyl) urea, commonly known as diazolidinyl urea, available under the trade name Germall® II from Sutton Laboratories, Inc.; N,N"-methylenebis{N'-[1-(hydroxymethyl)-2,5-dioxo-4-imidazolidinyl]urea}, commonly known as imidazolidinyl urea, available, e.g., under the trade name Abiol® from 3V-Sigma, Unicide U-13® from Induchem, Germall 115® from Sutton Laboratories, Inc.; polymethoxy bicyclic oxazolidine, available under the trade name Nuosept® C from Hüls America; formaldehyde; glutaraldehyde; polyaminopropyl biguanide, available under the trade name Cosmocil CQ® from ICI Americas, Inc., or under the trade name Mikrokill® from Brooks, Inc; dehydroacetic acid; and benzsiothiazolinone available under the trade name Koralone™ B-119 from Rohm and Hass Corporation.

Suitable levels of preservative are from about 0.0001% to about 0.5%, alternatively from about 0.0002% to about 0.2%, alternatively from about 0.0003% to about 0.1%, by weight of the composition.

The deodorant compositions herein may be in any suitable form, including, for example, roll-on deodorants, gel sticks or body sprays.

### II. Method of Making

The deodorant composition can be made in any suitable manner known in the art. For example, base spray and roll on chassis formulations can be prepared as pre-mixes (i.e. all of the ingredients except for the malodor polymer or complex are added to a vessel, stirred, and heated if necessary) and either the PVam polymer alone or as a metalized PVam polymer complex, can be added to a pre-mix.

### III. Methods of Use and Methods of Reducing Body Malodor

Deodorant compositions can be used, for example, by rubbing the composition on the skin via an applicator or by spraying the composition on the skin. Application can be achieved by using a spray device, a roller, a pad, a nozzle, etc. A method of use could be, for example, applying to a user a leave-on deodorant composition comprising an effective amount of a malodor control polymer comprising polyvinylamine. Another method of use could include applying to a user a leave-on deodorant composition comprising an effective amount of a metalated malodor control polymer comprising a water-soluble metal ion and a polyvinylamine polymer.

Also included herein are methods for reducing body malodor by reducing the population of bacteria in the underarm. A method for reducing body malodor can include, for example, applying a leave-on deodorant composition comprising an effective amount of a malodor control polymer comprising polyvinylamine,water-soluble metal ion and polyvinylamine, or a combination thereof; to at least a portion of the underarm of a user.

Another method includes a method of reducing underarm bacteria, comprising: applying a leave-on deodorant composition comprising a malodor control polymer comprising a polyvinylamine polymer, a water soluble metal ion and a polyvinylamine polymer, or a combination thereof, to at least a portion of the underarm of a user. Whether underarm bacteria are reduced can be determined by the Hair Pluck method.

The composition may comprise from about 0.01% to about 20%, by weight of the composition, of the malodor control polymer. In a further example, the composition may comprise from about 1.0 % to about 20%, by weight of the composition, of the malodor control polymer. The polyvinyl amine polymer may comprise a copolymer comprising about 95% mol vinyl monomer and about 5% mol vinylformamide monomer.

While some compositional components are listed in the methods section for illustration the deodorant compositions in the methods can contain any combination of components as discussed above in the Deodorant Composition section.

### IV. Test Methods

### A. In Vitro Inhibition of Odor Causing Bacteria - Minimal Inhibitory Concentration (MIC) Method:

Overnight cultures of clinically isolated strains of Corynebacteria mucifaciens or Staphylococcus epidermidis (obtained from the underarms of volunteer subjects) were created by using a single colony from a streak plate to inoculate 50 mL of Muller-Hinton + 1% Tween-80 (MHT) broth. Cultures were incubated overnight ∼18-22 hrs in a shaking incubator (33°C, 200rpm). A 1:1000 dilution of the respective culture was made in MHT broth and placed on ice. The 1:1000 inoculums were quantitated by plating onto MHT-agar to monitor the number of colony forming units (CFUs) added to the study plate(s). Plates were incubated 24 hrs (S. epidermidis) or 48 hrs (C. mucifaciens) at 33oC. Typically 105-106 CFUs (S. epidermidis) or 104- 105 CFUs (C. mucifaciens) were found to be added to each assay well.

Test compositions were assayed in triplicate employing 1:2 serial dilutions using MHT broth for a volume of 100 µl per well in a 96-well flat bottom culture plate. Once study materials were dispensed, 100 µl per well of the appropriate 1:1000 bacterial culture was used to inoculate. The assay plates were placed onto a plate shaker (∼500rpm) and incubated at 33oC for 24-28 hrs. The MIC was determined by visualization. Briefly, the lowest dose that remains transparent after serial dilution was determined to be the MIC value (100% inhibition of bacteria growth) for a given study material. The greater the dilution required to see the onset of bacterial growth the more potent the antibacterial activity of the test material.

### B. Hair Pluck and Self Assessed Body Odor Clinical Methods and Results

Individuals were subject to a 3-day washout period where no antiperspirant or deodorant was used and only soap and shampoo usage was permitted. Following the 3-day washout period a 10-day treatment study followed. For 10 consecutive days, the individual's underarm was limited to one treatment per day with the given tested trial product, and the individual was not permitted to use any antiperspirant or deodorant product. The dose of the tested trial product (e.g., leave on roll-on personal care composition) was limited to 500mg/underarm. Both the antibacterial activity (Hair Plucking) and anti-odor (Self Assessments) efficacy of the compositions were determined simultaneously in the same studies.

For Assessment of Antibacterial Activity: Replicate hairs were plucked and removed from the underarm at baseline (end of the washout period) and about 5 hours after the final (10^{th}) treatment and placed individually into separate Soleris vials. The vials were incubated at 37°C in a Soleris photometer and continuously monitored for 48 hours. Follicular associated bacteria present on the hair metabolize carbohydrates in the vials generating acid by products, decreasing the culture pH, and causing a color change in a pH indicator present in the vial. The time (hours) to color change, known as the detection time, is inversely proportional to the number and/or metabolic health of follicular bacteria carried over on the hair. That is, the longer the detection time the fewer in number or less "healthy" the follicular bacteria. Longer detection times are indicative of a strong antibacterial effect within the hair follicle while shorter times suggest a weak or non-existent antibacterial effect.

Figs. 1, 2, and 3 demonstrate the potent in vivo antibacterial efficacy of the PVam polymer alone and when metalated. In Figure 1 we can see the results from a clinical study (n = 21) evaluating the follicular antibacterial efficacy of a roll-on composition containing a metalated PVam polymer system (Lupamin® 1595 + ZnCl₂). In this study the metalated PVam composition provided significantly increased (p < 0.05) detection times from both the baseline value and in direct comparison to the control roll-on product at the end of the study. This indicates that the metalated PVam composition provided strong follicular antibacterial activity. In Figure 2 we can see the results from another clinical study (n = 19 subjects) evaluating the follicular antibacterial efficacy of roll-on products containing either the PVam polymer (Lupamin® 1595) alone or following metalation with ZnCl₂. As can be seen, both compositions significantly (p < 0.05) increased detection times from the baseline readings indicating a strong antibacterial effect following 10 days of product usage.

The results from a third clinical study (n = 22 subjects) are shown in Fig. 3 where a metalated PVam polymer system formulated in a base deodorant spray chassis demonstrated significantly superior antibacterial efficacy when compared to a base spray chassis containing zinc phenolsulfonate (ZPS - a commercially utilized deodorant antibacterial agent). Note that while a base spray chassis was used in this clinical study it was applied as a roll on, not as a spray. The results from both clinical studies clearly demonstrate a potent follicular antibacterial activity achieved following use of either a PVam or metalated PVam polymer.

For Assessment of Anti-Odor Efficacy: Subjects self assessed their underarm odor at baseline and twice/day (am & pm) throughout the clinical trial. For their individualized odor assessment, each subject removed their shirt, turned their head towards the underarm and sniffed. An odor score was assigned for each underarm using a 0 - 10 rating scale where 0 indicated no detectable odor and 10 represented the highest level of odor this subject had personally experienced on themselves. Underarm odor scores were designated as "12 hour" and "24 hour"; where "12 hour" scores represent odor measures taken in the evening, or about 12 hours after product use and "24 hour" scores represent odor measures taking the following morning, or about 24 hours after product use.

For clinical 1, Figs. 4 and 5 demonstrate the 12 and 24 hour odor protection achieved through use of the metalated PVam polymer system (Lupamin® 1595 + ZnCl₂) in a roll on chassis versus a control roll on chassis that does not contain the polymer system. As can be seen the metalated PVam composition provided a statistically significant (p < 0.05; repeated means) reduction in underarm odor at both 12 and 24 hours post use. Figs. 6 and 7 demonstrate the 12 and 24 hour odor protection achieved through use of the PVam only and metalated PVam polymer systems when formulated in roll-on chassis. As can be seen, at 12 and 24 hours post use, both compositions significantly (p < 0.05) reduced baseline odor over the course of the study. When compared against each other at 12 and 24 hours post product use, the PVam only composition was slightly more effective at odor control then the metalated PVam composition.

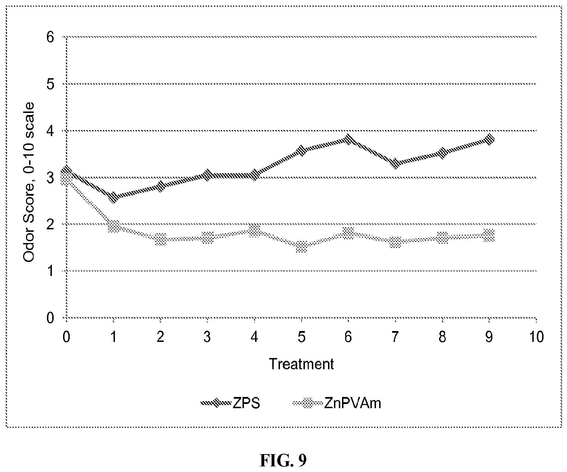

Figs. 8 and 9 demonstrate the superior 12 and 24 hour odor protection achieved through use of the metalated PVam polymer system (Lupamin® 1595 + ZnCl₂) in a base spray chassis versus a base spray chassis containing zinc phenolsulfonate (ZPS - a commercially utilized deodorant antibacterial agent). At both 12 and 24 hours after product use the metalated PVam composition provided a statistically significant (p < 0.05; repeated means) reduction in underarm odor versus the ZPS product.

### EXAMPLES

### Leave-on Deodorant Composition

Tables 2 and 3 show some non-limiting examples of inventive leave-on deodorant formulations.

**Table 2**

| | % Raw Material: | | |
|---|---|---|---|
| Ingredient | Example 1 Roll-On + Zn-PVam | Example 2 Roll-On + PVam | Example 3 Body Spray + PVam |
| Water | 79.25 | 81.50 | 76.40 |
| Arlamol E (emollient) | 3.00 | 3.00 | --- |
| Brij 72 (solubilizer/emulsifier) | 1.90 | 1.90 | --- |
| Brij 78 (solubilizer/emulsifier) | 1.10 | 1.10 | --- |
| Versene NA Crystals | 0.10 | 0.10 | --- |
| BHT (Antioxidant) | 0.05 | 0.05 | --- |
| PVam (Lupamin® 1595 - 16.2% active) | 12.35 | 12.35 | 12.35 |
| ZnCl2 (Zinc metal source) | 0.75 | --- | 0.75 |
| 0.1N HCl (acidify ZnCl2) | 1.50 | --- | 1.50 |
| Dipropylene Glycol (emollient) | --- | --- | 5.00 |
| Cremophor Rh 60 (solubilizer/surfactant) | --- | --- | 3.00 |
| Symdiol 68 (preservative) | --- | --- | 1.00 |
| Total % | 100.00 | 100.00 | 100.00 |

### Example 1: Roll-On Chassis + Zinc-PVam

Metalated polymer complex (Lupamin® 1595 complexed with ZnCl₂) was prepared in the following way: ZnCl₂ was dissolved in water and the resulting solution was acidified with 0.1N hydrochloric acid until the solution was clear. PVam was then added slowly and stirred at room temperature for a minimum of one hour. The resulting ZnPVam solution was then added to the pre-made roll-on base chassis.

Roll-On base chassis: Water was placed in an appropriate sized container, and then heated to 80-85°C with stirring. Once the temperature reached about 40°C, Versene NA2 was added into the solution. In a separate container, Arlamol E, Brij 72, Brij 78, and BHT (oil phase) were combined and heated (∼80°C) with stirring until the solution was clear. Once the water/Versene NA2 premix reached 80-85°C, the oil phase solution was slowly transferred into the water phase. The resulting combined solution was stirred for a minimum of 15 minutes. Following mixing the solution was cooled until the temperature reached between 50-70°C at which point the metalated polymer complex was slowly added. The full formulation was stirred while cooling to room temperature.

### Example 2: Roll On Chassis + PVam

The Roll-On base chassis was prepared as described above. When during cooling the temperature of the Roll On base chassis pre-mix reached between 30-50°C, the PVam was slowly added with stirring. The PVam Roll On solution was then continuously stirred while cooling to room temperature.

### Example 3: Spray Chassis + Zinc-PVam

Body Spray Base Chassis: Water was placed in an appropriate sized container. Cremophor was added with stirring until it was completely dissolved into the water. Dipropylene glycol and Symdiol 68 were then added to the water/Cremophor solution and stirred for 30 minutes at room temperature.

Zinc- Polymer complex (Lupamin® 1595 complexed with ZnCl2) was prepared separately as described in Example 1 and then slowly added to the body spray base chassis and stirred at room temperature for 30 minutes.

**Table 3**

| | % Raw Material | % Raw Material |
|---|---|---|
| Ingredient | Example 4 - Aerosol Body Spray | Example 5 - Aerosol Body Spray |
| Ethanol | 36.4 | 36.4 |
| Water | 31.55 | 22.54 |
| PVam (Lupamin® 1595 - 10.5% active) | 4.85 | 13.86 |
| Dimethyl Ether | 27.2 | 27.2 |

Examples 4 and 5 are made by combining the ethanol, water, and PVam in a vessel and mixing until homogeneous. This is the concentrate. The concentrate is then filled in a package suitable for a pressurized aerosol and filled with propellant at the noted amount.

Throughout this specification, components referred to in the singular are to be understood as referring to both a single or plural of such component.

All percentages stated herein are by weight unless otherwise specified.

Every numerical range given throughout this specification will include every narrower numerical range that falls within such broader numerical range, as if such narrower numerical range were all expressly written herein. For example, a stated range of "1 to 10" should be considered to include any and all subranges between (and inclusive of) the minimum value of 1 and the maximum value of 10; that is, all subranges beginning with a minimum value of 1 or more and ending with a maximum value of 10 or less, e.g., 1 to 6.1, 3.5 to 7.8, 5.5 to 10, etc.

Further, the dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. Use of a malodor control polymer comprising a polyvinyl amine polymer, wherein the polyvinyl amine polymer comprises a copolymer comprising 95% mol vinyl monomer and 5% mol vinylformamide monomer, in a cosmetic composition for reducing the odor caused by the growth of bacteria on skin, wherein the bacteria is C. mucifaciens.

2. Use of a malodor control polymer comprising a polyvinyl amine polymer, wherein the polyvinyl amine polymer comprises a copolymer comprising 95% mol vinyl monomer and 5% mol vinylformamide monomer, in a cosmetic composition for inhibiting the growth of odor-causing bacteria on skin, wherein the bacteria is C. mucifaciens.

3. The use of any preceding claim, wherein the malodor control polymer is complexed with zinc.

4. The use of claim 3, wherein the zinc to malodor control polymer ratio is from 0.001 to 50, preferably from 0.001 to 20, more preferably from 0.001 to 15, even more preferably from 0.001 to 10.

5. The use of any preceding claim, wherein the cosmetic composition comprises from 0.01% to 20%, preferably from 0.5 to 20%, more preferably from 1.0% to 15%, even more preferably from 2.0% to 10%, by weight of the composition, of the malodor control polymer.

6. The use of any preceding claim, wherein the cosmetic composition is a deodorant composition, preferably wherein the cosmetic composition is a deodorant composition to be applied onto the underarm.

7. The use of any preceding claim, wherein the cosmetic composition is in the form of a stick, a roll-on, or a spray.

8. The use of any preceding claim, wherein the cosmetic composition further comprises water, alcohol, and a propellant.

9. The use of claim 8, wherein the alcohol comprises ethanol.

10. The use of claims 8 or 9, wherein the propellant comprises dimethyl ether.

11. The use of any preceding claim, wherein the composition further comprises water and an emollient.

12. The use of any preceding claim, wherein the malodor control polymer is 95% hydrolyzed.

## Patentansprüche

1. Verwendung eines Polymers zum Bekämpfen von üblen Gerüchen, umfassend ein Polyvinylaminpolymer, wobei das Polyvinylaminpolymer ein Copolymer umfasst, das zu 95 Mol-% Vinylmonomer und zu 5 Mol-% Vinylformamidmonomer umfasst, in einer kosmetischen Zusammensetzung zur Minderung des Geruchs, der durch das Wachstum von Bakterien auf Haut verursacht wird, wobei das Bakterium C. mucifaciens ist.

2. Verwendung eines Polymers zum Bekämpfen von üblen Gerüchen, umfassend ein Polyvinylaminpolymer, wobei das Polyvinylaminpolymer ein Copolymer umfasst, das zu 95 Mol-% Vinylmonomer und 5 Mol-% Vinylformamidmonomer umfasst, in einer kosmetischen Zusammensetzung zur Hemmung des Wachstums Geruch verursachender Bakterien auf Haut, wobei das Bakterium C. mucifaciens ist.

3. Verwendung nach einem der vorstehenden Ansprüche, wobei das Polymer zum Bekämpfen von üblen Gerüchen mit Zink komplex gebunden wird.

4. Verwendung nach Anspruch 3, wobei das Verhältnis von Zink zu üble Gerüche bekämpfendem Polymer von 0,001 bis 50, vorzugsweise von 0,001 bis 20, mehr bevorzugt von 0,001 bis 15, noch mehr bevorzugt von 0,001 bis 10 beträgt.

5. Verwendung nach einem der vorstehenden Ansprüche, wobei die kosmetische Zusammensetzung von 0,01 Gew.-% bis 20 Gew.-%, vorzugsweise von 0,5 bis 20 Gew.-%, mehr bevorzugt von 1,0 Gew.-% bis 15 Gew.-%, noch mehr bevorzugt von 2,0 Gew.-% bis 10 Gew.-% der Zusammensetzung das üble Gerüche bekämpfende Polymer umfasst.

6. Verwendung nach einem der vorstehenden Ansprüche, wobei die kosmetische Zusammensetzung eine Deodorantzusammensetzung ist, wobei die kosmetische Zusammensetzung vorzugsweise eine auf die Achselhöhle aufzutragende Deodorantzusammensetzung ist.

7. Verwendung nach einem der vorstehenden Ansprüche, wobei die kosmetische Zusammensetzung in der Form eines Sticks, Deorollers oder Sprays vorliegt.

8. Verwendung nach einem der vorstehenden Ansprüche, wobei die kosmetische Zusammensetzung ferner Wasser, Alkohol und ein Treibmittel umfasst.

9. Verwendung nach Anspruch 8, wobei der Alkohol Ethanol umfasst.

10. Verwendung nach einem der Ansprüche 8 oder 9, wobei das Treibmittel Dimethylether umfasst.

11. Verwendung nach einem der vorstehenden Ansprüche, wobei die Zusammensetzung ferner Wasser und einen Weichmacher umfasst.

12. Verwendung nach einem der vorstehenden Ansprüche, wobei das Polymer zur Bekämpfung von üblen Gerüchen zu 95 % hydrolysiert ist.

## Revendications

1. Utilisation d'un polymère de lutte contre les mauvaises odeurs comprenant un polymère polyvinylamine, dans laquelle le polymère polyvinylamine comprend un copolymère comprenant 95 % molaires de monomère vinylique et 5 % molaires de monomère vinylformamide, dans une composition cosmétique pour réduire l'odeur provoquée par la croissance de bactéries sur la peau, dans laquelle les bactéries sont C. mucifaciens.

2. Utilisation d'un polymère de lutte contre les mauvaises odeurs comprenant un polymère polyvinylamine, dans laquelle le polymère polyvinylamine comprend un copolymère comprenant 95 % molaires de monomère vinylique et 5 % molaires de monomère vinylformamide, dans une composition cosmétique pour inhiber la croissance de bactéries responsables d'odeurs sur la peau, dans laquelle les bactéries sont C. mucifaciens.

3. Utilisation selon une quelconque revendication précédente, dans laquelle le polymère de lutte contre les mauvaises odeurs est complexé avec du zinc.

4. Utilisation selon la revendication 3, dans laquelle le rapport du zinc au polymère de lutte contre les mauvaises odeurs va de 0,001 à 50, de préférence de 0,001 à 20, plus préférablement de 0,001 à 15, même plus préférablement de 0,001 à 10.

5. Utilisation selon une quelconque revendication précédente, dans laquelle la composition cosmétique comprend de 0,01 % à 20 %, de préférence de 0,5 à 20 %, plus préférablement de 1,0 % à 15 %, même plus préférablement de 2,0 % à 10 %, en poids de la composition, du polymère de lutte contre les mauvaises odeurs.

6. Utilisation selon une quelconque revendication précédente, dans laquelle la composition cosmétique est une composition déodorante, de préférence dans laquelle la composition cosmétique est une composition déodorante à appliquer sur l'aisselle.

7. Utilisation selon une quelconque revendication précédente, dans laquelle la composition cosmétique est sous la forme d'un bâton, d'un applicateur à bille ou d'une pulvérisation.

8. Utilisation selon une quelconque revendication précédente, dans laquelle la composition cosmétique comprend en outre de l'eau, de l'alcool et un propulseur.

9. Utilisation selon la revendication 8, dans laquelle l'alcool comprend de l'éthanol.

10. Utilisation selon la revendication 8 ou 9, dans laquelle le propulseur comprend de l'éther diméthylique.

11. Utilisation selon une quelconque revendication précédente, dans laquelle la composition comprend en outre de l'eau et un émollient.

12. Utilisation selon une quelconque revendication précédente, dans laquelle le polymère de lutte contre les mauvaises odeurs est hydrolysé à 95 %.
